# EUROPEAN PATENT SPECIFICATION

(11) **EP 1 052 970 B1**
(45) Date of publication and mention of the grant of the patent: **02.05.2003**
(21) Application number: 99906922.2
(22) Date of filing: 08.02.1999
(51) Int. Cl.: A61K 7/48, A61K 7/06

(54) **RESIN COMPOSITION FOR COSMETICS**
HARZZUSAMMENSETZUNG FÜR KOSMETIKA
COMPOSITION A BASE DE RESINE A USAGE COSMETIQUE

(30) Priority: 09.02.1998 JP 2759598
(43) Date of publication of application: 22.11.2000
(73) Proprietor: National Starch and Chemical Investment Holding Corporation, Wilmington, Delaware 19803-7663 (US)
(72) Inventor: KOYAMA, Katsuya, Itami, Hyogo 664-0898 (JP); TSUZUKI, Toshitaka, Osaka 562-0036 (JP); ASAOKA, Seiji, Osaka 565-0841 (JP); SAKURAI, Takahiro, Osaka 562-0036 (JP)
(74) Representative: Held, Stephan, Dr.rer.nat., Dipl.-Chem.
(86) International application number: US9902960
(87) International publication number: WO99039688

(56) References cited:
- EP-A- 0 619 111
- EP-A- 0 636 361
- EP-A- 0 773 246
- EP-A- 0 838 212
- WO-A-94/13724
- DE-A- 4 225 045
- DE-A- 19 541 326

## Description

The present invention relates to aqueous cosmetic resin compositions which comprise as a major ingredient an amphoteric urethane resin and in particular to those compositions for use in skin-care or hair-care products as hairstyling compositions, conditioning agents, or viscosity-regulating agents.

Conventionally, base resins such as anionic acrylic resins, anionic/cationic amphoteric acrylic resins, or nonionic polyvinylpyrrolidone resins are used in hairstylings. Such base resins, however, have drawbacks that they have poor feeling and combability, and also result in flaking, although they exhibit an excellent setting ability for fixedly styling the hair. On the other hand, when the feeling and combability are put above other properties, different problems such as a poor setting ability will rise. It is thus difficult to have all the characteristics required for hairstylings regarding the setting ability, feeling, combability and anti-flaking property, when the conventional resins as described above are used.

In order to solve the problems above, use of anionic urethane resins instead of the acrylic or other resins above has been proposed (Japanese Patent Publication (Kokai) No. H6 (1994)-321741). When used as a base resin for a hairstyling, such a urethane resin allows the hairstyling to have conflicting properties such as a good setting ability, good feeling, good combability, and good anti-flaking property. Such an anionic urethane resin, however, cannot be washed away satisfactorily with a shampoo or the like, and thus have a drawback in its poor washability.

Patent document EP-A-0 619 111 discloses a hair fixative composition containing low amounts of volatile organic solvent, formulated with a soluble polyurethane prepared from an organic diisocyante, a diol, and a 2,2-hydroxymethyl-substituted carboxylic acid neutralized with a cosmetically acceptable organic or inorganic base.

Patent document DE 195 41 326 discloses water-soluble or water-dispersible polyurethanes of a water-dispersible polyurethane prepolymer having terminal isocyanate groups and a primacy or secondary amine having at least one ioriogenic or ionic group and salts thereof. These polyurethanes can be used as auxiliaries in cosmetology and pharmacy and, in particular, as hair lacquers having enhanced ease of washoff.

In light of such circumstances, the present invention aims to provide a cosmetic resin composition which has both of a sufficient adhesive property to the hair and a sufficient washability without adversely affecting properties required for the hairstyling regarding the setting ability, feeling, combability, and anti-flaking property.

In order to achieve the above aim, the cosmetic resin composition of the present invention comprises as a major ingredient an amphoteric urethane resin having one or more carboxyl groups and one or more tertiary groups in a single molecule

The present inventors have concentrated their efforts in research for base resins which have all the properties required for the hairstylings regarding the setting ability, the feeling, combing property, anti-flaking property, adhesive property to the hair, and washability. As a consequence, the present inventors have finally found that such an aim can be achieved by the use of a resin composition which comprises as a major ingredient the amphoteric urethane resin having both carboxyl groups and tertiary amino groups in a single molecule. The present invention has been thus completed on the basis of this finding. Specifically, when a urethane resin is used as a main structure of the base resin, the desirable setting ability can be attained because of elasticity and toughness of the urethane resin, simultaneously with the feeling, combability, and anti-flaking property which are all intrinsically conflicting with the setting ability. We have found, however, that such a urethane resin cannot be easily washed away from the hair when used in the hairstyling, because the molecular chains of the urethane resin are prone to intertangle with each other and thereby the form films, and that when the washability is preferential, the elasticity and toughness of the urethane resin are impaired. After our further intensive efforts to solve such problems, we have found that by using the amphoteric urethane resins having both of carboxyl groups and tertiary amino groups, it becomes possible to produce a hairstyling which is highly water-resistant in neutral water because of ionic bonds between the carboxyl groups and tertiary amino groups, and which also exhibits excellent washability because of the cleavage of such ionic bonds when washed with shampoos or the like. Furthermore, the amophoteric urethane resin above has a better adhesive property compared to the conventional anionic urethane resin, since the cationic tertiary amino groups contained in the molecular chain interact with the negatively charged surface of the hair.

In addition, a particularly good washability can be achieved by adjusting a ratio of the carboxyl groups to the tertiary amino groups (a ratio between the numbers of respective functional groups) in the amphoteric urethane resin within a certain range.

Embodiments for carrying out the present invention are described below.

The cosmetic resin composition of the present invention comprises as a major ingredient the amphoteric urethane resin having the carboxyl groups and tertiary amino groups in a single molecule. Such a cosmetic resin composition is used in skin-care or hair-care products as a hairstyling agent, conditioning agent, or viscosity-regulating agent, and preferably as a hairstyling agent. A composition "comprising as a major ingredient the amphoteric urethane resin" generally intends to mean that the composition also contains other ingredient(s) in addition to the resin, but the phrase is also intended to include a composition which consists of only the amphoteric urethane resin.

The amphoteric urethane resin as described above may be prepared, for example, by reacting a polyol compound (Component A), a polyisocyanate compound (Component B), and a compound having one or more active hydrogens and one or more carboxyl groups (Component C) under the excessive presence of isocyanate groups to prepare a prepolymer containing the isocyanate groups, and then reacting the prepolymer containing the isocyanate groups with a compound having one or more active hydrogens and one or more tertiary amino groups (Component D). Alternatively, the reaction sequence of Components C and D may be exchanged, that is the amphoteric urethane resin as described above may also be prepared by reacting Components A, B and D under the excessive presence of isocyanate groups to prepare a prepotymer containing the isocyanate groups, and then reacting the prepolymer containing the isocyanate groups with particular Component C. According to such processes, the amphoteric urethane resin can be produced more easily and more safely compared to the conventional processes. In the processes above, an intended amphoteric urethane resin cannot be obtained when Component C and Component D are simultaneously reacted with Components A and B, because in such a procedure, the carboxyl groups in Component C firstly form salts with the tertiary amino groups in Component D to become insoluble in the reaction system, and thereby precluding their reactions with the isocyanate compound in spite of the presence of OH groups. Thus, the desired amphoteric urethane resin can be produced as described above by reacting Components A and B with one of Components C and D, and thereafter reacting with the other component.

The polyol compound used as Component A above is not specifically restricted so far as it is generally used in the production of the general polyurethane, and includes, for example, polyester polyols, polyether polyols, polycarbonate polyols, polybutadiene polyols, polyisoprene polyols, polyolefin polyols, and polyacrylate polyols. Such polyol compounds are used alone or in combination with one or more other such compounds. Among them, the polyester polyols and polyether polyols are particularly preferred. Examples of such a polyester polyol are those obtained by polycondensation of at least one of dicarboxylic acids such as succinic acid, glutaric acid, adipic acid, sebacic acid, azelaic acid, maleic acid, fumaric acid, phthalic acid, and terephthalic acid with at least one of polyols such as ethylene glycol, propylene glycol, 1,4-butanediol, 1,3-butanediol, 1,6-hexanediol, neopentyl glycol, 1,8-octanediol, 1,10-decanediol, diethylene glycol, spiroglycol, and trimethylolpropane, as well as those obtained by ring-opening polymerization of lactones. Examples of such a polyether polyol which may be used are those obtained by ring-opening addition polymerization of cyclic ethers such as ethylene oxide, propylene oxide, oxetane, and tetrahydrofuran with water or polyols used in synthesis of said polyester polyols, or with phenols such as bisphenol A, or primary or secondary amines. Specific examples of such polyether polyol are polyoxyethylene polyols, polyoxypropylene polyols, polyoxytetramethylene polyols, those obtained by ring-opening addition polymerization of at least one of propylene oxide and ethylene oxide with bisphenol A (in the case of a copolymer, it may be a block or a random copolymer).

The polyisocyanate compound used as Component B above is not specifically restricted, and includes, for example, organic diisocyanate compounds such as aliphatic diisocyanate, alicyclic diisocyanate, aromatic diisocyanate compounds. Such polyisocyanate compounds are used alone or in combination with one or more other such compounds. Examples of the aliphatic diisocyanate compound includes ethylene diisocyanate, 2,2,4-trimethylhexamethylene diisocyanate, and hexamethylene-1,6-diisocyanate. Examples of the alicyclic diisocyanate compound includes hydrogenated diphenylmethane-4,4'-diisocyanate, cyclohexane-1,4-diisocyanate, methylcyclohexylene diisocyanate, isophorone diisocyanate and norbornane diisocyanate. Examples of the aromatic diisocyanate includes diphenylmethane-4,4'-diisocyanate, xylylene diisocyanate, toluene diisocyanate, and naphthalene diisocyanate. Among these compounds, hexamethylene-1,6-diisocyanate, isophorone diisocyanate, and norbornane diisocyanate are preferred in view of their low costs.

The compound having one or more active hydrogens and one or more carboxyl groups, used as Component C, is not specifically restricted so far as it has at least one active hydrogen and at least one carboxyl group in the molecule, and includes, for example, dimethylolpropionic acid (DMPA), dimethylolbutanoic acid, and polycaprolactonediol containing carboxyl group(s).

The compound having one or more active hydrogens and one or more tertiary amino groups, used as Component D, is not specifically restricted so far as it has at least one active hydrogen and at least one tertiary amino group in the molecule, and includes, for example, N-alkyldialkanolamine compounds such as N-methyldiethanolamine and N-butyldiethanolamine, as well as dimethylaminoethanol.

In the preparation of the prepolymers containing the isocyanate groups by using the components above, it is preferred to use a chain-lengthening agent. It becomes possible by using the chain-lengthening agent to regulate various properties of the amphoteric urethane resin obtained as a final product. The chain-lengthening agent which may be used is not specifically restricted, and includes low molecular weight polyols, amines, and the like. Examples of such a low molecular weight polyol are glycols such as ethylene glycol, propylene glycol, 1,4-butanediol, diethylene glycol, 1,6-hexenediol, spiroglycol, bis(β-hydroxyethoxy)benzene, and xylylene glycol, as well as triols such as trimethylolpropane and glycerin. An example of the amine used for that purpose is methylenebis(o-chloraniline).

It is preferred for all of the polyol compound (Component A), the polyisocyanate compound (Component B), the compound having one or more active hydrogens and one or more carboxyl groups (Component C), the compound having one or more active hydrogens and one or more tertiary amino groups (Component D), and the chain-lengthening agent described above to be polyfunctional compounds having 3 or more different functions, since use of such polyfunctional compounds results in improvement of physical properties such as heat-resistance of the polyurethane-urea polymers by formation of cross-links.

In production of the amphoteric urethane resins, a solvent may be optionally used, and it is particularly preferred to use a solvent in which both of the raw materials and the formed polyurethane are soluble. Examples of such a solvent are amides such as N-methylpyrrolidone, dimethylformamide, and dimethylacetamide, ketones such as acetone and methyl ethyl ketone, and esters such as ethyl acetate, as well as cellosolve acetates and cellosolve ethers. In addition, a polymerization catalyst well known in the art of the polyurethanes such as tertiary amine catalyst are diazabicyclo[2.2.2]octane (DABCO), tetramethylenediamine, N-methylmorpholine, and diazabicycloundecene (DBU). An example of the organometalic catalyst is dibutyltin dilaurate.

A preferred amphoteric urethane resin prepared from the components above has a ratio of the carboxyl groups to the tertiary amino groups (a ratio between the numbers of respective functional groups) which has been set at carboxyl group/tertiary amino group = 1/10 to 10/1. This is because the amphoteric urethane resins in which the ratio of the carboxyl groups to the tertiary amino groups has been set in the range above has a good washability.

The amphoteric urethane resin described above is dispersed in water to obtain an aqueous dispersion or solubilized in water to obtain an aqueous solution for the use in a skin-care or a hair-care product as a hairstyling agent, conditioning agent, or viscosity-regulating agent. In this case, it is preferred to disperse the amphoteric urethane resin in water containing an aliphatic polyamine. This is because when the excessive isocyanate groups are used in the polymerization while keeping the polymerization degree low and the polymer is reacted with the aliphatic polyamine while being dispersed in water, the NH and/or NH2 groups of the aliphatic polyamine rapidly react with the isocyanate groups, which allows the polymerization to proceed in water while forming urea bonds. Such an aliphatic polyamine is not specifically restricted, and includes, for example, triethylamine, ethylenediamine, propylenediamine, piperazine, diethylenetriamine, and the like. It is noted that a silane coupling agent may also be added as an additive to said aqueous dispersions of the amphoteric urethane resin so as to improve their adhesion to other substrate. Furthermore, various other additive(s) such as a protective colloid an antibacterial agent or an antifungal agent may also be added to improve the storage stability.

The aqueous dispersion or aqueous solution of the amphoteric urethane resin thus prepared is used as a hair gel composition in combination with, for example, a thickening agent, ethanol, and deionized water. Alternatively, such an aqueous dispersion or aqueous solution of the amphoteric urethane resin is used as hair foam composition in combination with, for example, various surfactants such as polyoxyethylene alkyl ether and a palm-oil fatty acid diethanolamide, a propellant (LPG), dimethyl ether, ethanol, and other various additives (a silicon-based compound, an amine-based compound).

The followings are descriptions of working examples and a comparative example.

### Example 1

Into a four-necked glass flask attached with a stirrer, thermometer, and nitrogen inlet tube, 100 g of isophorone diisocyanate (IPDI), 225 g of polyoxytetramethylene glycol (PTMG, MW = 2000), and 18 g of dimethylolpropionic acid (DMPA) were introduced, and 30 g of ethyl acetate as organic solvent and 0.02 g of dibutyltin dilaurate as a catalyst were added thereto. The mixture was then heated to 80°C using an oil bath, and the reaction was allowed to proceed for 4 hours while maintaining that temperature. The temperature was then lowered to 70°C, and 8 g of N-methyldiethanolamine (NMDEA) and 30 g of ethyl acetate were further added. The reaction was allowed to proceed for additional 2 hours to obtain a solution of a polyurethane prepolymer having remaining NCO groups. This polyurethane prepolymer having the remaining NCO groups was dispersed in 600 g of water containing 12 g of triethylamine, and chain-lengthening reaction was allowed to proceed at 50°C for 3 hours to convert the prepolymers to higher molecular weight polymers. The ethyl acetate was recovered from the resultant aqueous dispersion to obtain an aqueous dispersion of the amphoteric urethane resin which substantially contained no organic solvent.

### Example 2

An aqueous dispersion of an amphoteric urethane resin was obtained as described in Example 1 except that the amount of the N-methyldiethanolamine (NMDEA) was changed to 4 g.

### Example 3

An aqueous dispersion of an amphoteric urethane resin was obtained as described in Example 1 except that the amount of N-methyldiethanolamine (NMDEA) was changed to 13 g.

### Comparative Example

An aqueous dispersion of an anionic urethane resin was obtained as described in Example 1 except that N-methyldiethanolamine (NMDEA) was not used.

The aqueous dispersions thus obtained in Examples 1-3 and Comparative Example were used at blending ratios described below in the preparation of Hair Gel Compositions "a" - "d".

### Hair Gel Composition "a"

To obtain component X, the ingredients shown below in Table 1 were blended with each other at the ratio indicated therein, and mixed until a viscous gel was obtained. The component Y which was obtained by mixing the ingredients shown in Table 1 at the ratio indicated therein was then added to the component X above, and gently mixed to homogeneity so as to obtain Hair Gel Composition "a".

**Table 1**

| Hair Gel Composition "a" | | |
|---|---|---|
| | | (Parts by weight) |
| Component X | Thickening agent *1 | 1.5 (Dry weight) |
| | Triethanolamine | 1.1 |
| | Ethanol | 10.0 |
| | Deionized water | 50.0 |
| Component Y | Aqueous dispersion of Example 1 | 3.0 (Dry weight) |
| | Deionized water | 34.4 |

| | | |
|---|---|---|
| *1:Structure 2001 (National Starch and Chemical Company) | | |

### Hair Gel Compositions "b" and "c"

Hair Gel Compositions "b" and "c" were prepared according to the procedure described above for Hair Gel Composition "a", except that the aqueous dispersions of Examples 2 and 3 were each used in Component Y shown above in Table 1 instead of the aqueous dispersion of Example 1.

### Hair Gel Composition of "d"

Hair Gel Composition "d" was prepared according to the procedure described above for Hair Gel Composition "a", except that the water dispersion of Comparative Example was used in Component Y shown above in Table 1 instead of the water dispersion of Example 1.

Hair Gel Compositions "a" - "d" thus obtained were used to evaluate their washability according to criteria described below. The results are shown in Table 2.

### Washability

Each Hair Gel Composition was applied on a glass plate to form a film 40 µm thick, and the film was then washed with a commercially available shampoo solution. The washability thus evaluated is indicated according to the following criteria: ⊗, the film could be washed away quite easily; ⊕, the film could be washed away easily; x, the film could not be washed away sufficiently.

**Table 2**

| Washability | |
|---|---|
| Hair Gel Composition "a" | ⊗ |
| Hair Gel Composition "b" | ⊕ |
| Hair Gel Composition "c" | ⊕ |
| Hair Gel Composition "d" | × |

It is apparent from the results shown in Table 2 that all Hair Gel Compositions "a" - "c" employing the amphoteric urethane resins of Examples 1-3, respectively, have excellent washability, and that Hair Gel Composition "a" which employed the amphoteric urethane resin of Example 1 has particularly good washability. This is probably because the ionic bonds between the carboxy group and the tertiary amino group in the amphoteric urethane resin are cleaved with the shampoo solution. On the contrary. Hair Gel Composition "d" which employed the anionic urethane resin of Comparative Example had quite poor washability, and thus was unsuitable for use as the hairstyling.

The feeling, setting ability, combability, anit-flaking property, and antistatic property were then evaluated using Hair Gel Compositions "a" - "d" according to the criteria described below. The results are shown below in Table 3. Specifically, 1.0 g of each Hair Gel Composition was separately applied to a bundle of black virgin hair (length: 15 cm, weight: 3.0 g), and evaluated for the properties above after drying.

### Feeling

A hairstyling which resulted in the hair having excellent feeling or touch after drying is indicated by ⊕.

### Setting Ability

A hairstyling which had an excellent setting ability without resulting in the tousled hair after drying is indicated by ⊕.

### Combability

A hairstyling which resulted in the hair being easily combed after drying is indicated by ⊕.

### Anti-flaking property

A hairstyling which resulted in the hair having no bloom on it at all after combing 10 times at dryness is indicated by ⊗, while a hairstyling which resulted in the hair having only slight bloom is indicated by ⊕.

### Antistatic property

A hairstyling which generated no static electricity at all after combing 10 times at dryness in indicated by ⊗, while a hairstyling which generated only slight static electricity is indicated by ⊕.

**Table 3**

| | Feeling | Setting ability | Combability | Anti-flaking property | Anti-static property |
|---|---|---|---|---|---|
| Hair Gel Composition "a" | ⊕ | ⊕ | ⊕ | ⊗ | ⊗ |
| Hair Gel Composition "b" | ⊕ | ⊕ | ⊕ | ⊗ | ⊗ |
| Hair Gel Composition "c" | ⊕ | ⊕ | ⊕ | ⊗ | ⊗ |
| Hair Gel Composition "d" | ⊕ | ⊕ | ⊕ | ⊕ | ⊕ |

As apparent from the results shown in Table 3, Hair Gel Compositions "a" - "c" which employed the amphoteric urethane resins of Examples 1-3, respectively, gave similar or even superior results for all the properties, compared to Hair Gel Composition "d" which employed the anionic urethane resin of Comparative Example. In particular, it is shown that Hair Gel Compositions "a" - "c" have excellent anti-flaking and anti-static properties.

In another experiment, Hair Foam Compositions "a" - "d" were prepared using the water dispersions of Examples 1-3 and Comparative Example at the ratios indicated below.

### Hair Foam Composition "a"

To obtain the Component X, the ingredients shown below in Table 4 were combined with each other at the ratio indicated therein, and mixed to homogeneity. The Component Y shown in Table 4 was then added to the Component X to obtain Hair Foam Composition "a".

**Table 4**

| Hair Foam Composition "a" | | |
|---|---|---|
| | | (Parts by weight) |
| Component X | Aqueous dispersion of Example 1 | 5.0 (Dry weight) |
| | Deionized water | 75.7 |
| | Polyoxyethylene stearyl ether *1 | 0.5 |
| | Ethanol | 10.0 |
| | Palm oil fatty acid diethanolamide *2 | 0.8 |
| Component Y | Propellant (LPG) | 8.0 |

| | | |
|---|---|---|
| *1: NIKKOL BS-20 (Nikko Chemicals Co.) | | |
| *2: Amycol CDE-1 (Miyoshi Yushi Co.) | | |

### Hair Foam Compositions "b" and "c"

Hair Foam Compositions "b" and "c" were prepared according to the procedure described above for Hair Foam Composition "a", except that the aqueous dispersions of Examples 2 and 3 were each used in the Component X shown above in Table 4 instead of the aqueous dispersion of Example 1.

### Hair Foam Composition "d"

Hair Foam Composition "d" was prepared according to the procedure described above for Hair Foam Composition "a", except that the aqueous dispersion of Comparative Example was used in the Component X shown above in Table 4 instead of the aqueous dispersion of Example 1.

Hair Foam Compositions "a" - "d" thus obtained were used to evaluate their washability, feeling, setting ability, combability, anti-flaking property, and anti-static property, according to the criteria described above. The results of such evaluations were nearly the same as those obtained for Hair Gel Compositions "a" - "d". Thus, all Hair Foam Compositions "a" - "c" which employed the amphoteric urethane resins of Example 1-3, respectively, had good washability, and exhibited similar or even superior properties also in other evaluations, compared to Hair Foam Composition "d" which employed the anionic urethane resin of Comparative Example.

In addition to the hairstylings described above, the cosmetic resin compositions of the present invention may be used in, for example, conditioning shaving creams and skin-care lotions.

### Conditioning shaving cream

To obtain the Component X, the ingredients shown below in Table 5 were mixed at the ratio indicated therein, and heated to 80°C. Separately, the Component Y was obtained by mixing the ingredients shown in Table 5 at the ratio indicated therein and heating the mixture to 80°C. The Components X and Y thus obtained were then mixed at 80°C, and cooled to 40°C. To the cooled mixture, appropriate amounts of an antiseptic agent and a perfume were added to obtain an intended conditioning shaving cream.

**Table 5**

| | | (% by weight) |
|---|---|---|
| Component X | Stearic acid | 8.00 |
| | Mineral oil | 2.00 |
| | Isopropyl myristate | 2.00 |
| | Glyceryl stearate | 0.50 |
| Component Y | Deionized water | 81.60 |
| | Triethanolamine (99%) | 4.20 |
| | Aqueous dispersion of Example 1 | 1.70 |

### Skin-care lotion

To obtain the Component X, the ingredients shown below in Table 6 were mixed at the ratio indicated therein, and heated to 80°C. Separately, the Component Y was obtained by mixing the ingredients shown in Table 6 at the ratio indicated therein and heating the mixture to 80°C. The Components X and Y thus obtained were then mixed and stirred at 80°C for 30 minutes. To the mixture, 20% by weight of 2% Carbopol 940 in water was then added, and stirred to homogeneity. The mixture was then cooled to 40°C to obtain an intended skin-care lotion.

**Table 6**

| | | (% by weight) |
|---|---|---|
| Component X | Octyl methoxycinnamate | 7.50 |
| | Polyoxy (PO) stearate ether | 1.00 |
| | Emulsified glyceryl stearate | 1.00 |
| | Stearic acid | 1.50 |
| | Titanium dioxide/C₁₂-C₁₅ alkyl benzoate (mixture) | 1.70 |
| | Polyoxyethylene (POE)-added Dimethicone | 0.50 |
| Component Y | Deionized water | 59.47 |
| | Triethanoiamine (99%) | 4.00 |
| | Aqueous dispersion of Example 1 | 3.33 |
| | Antiseptic agent | Appropriate amount |

### Effects of the Invention

As described above, the cosmetic resin composition of the present invention comprises as a major ingredient the amphoteric urethane resin having the carboxyl groups and tertiary amino groups, and thereby have all the properties required for the hairstylings regarding the setting ability, feeling, combability, anti-flaking property, adhesive property to hair and washability. Specifically, when a urethane resin is used as a main structure of the base resin, the desirable setting ability can be attained because of the elasticity and toughness of the urethane resin, simultaneously with the feeling, combability, and the anti-flaking property which are all intrinsically conflicting with the setting ability. By using the amphoteric urethane resins having both of carboxyl groups and tertiary amino groups, it becomes possible to produce a hairstyling which is highly water-resistant in neutral water because of ionic bonds between the carboxyl groups and the tertiary amino groups, and which also exhibits excellent washability because of the cleavage of such ionic bonds when washed with shampoos or the like. Furthermore, the amphoteric urethane resin above nas a better adhesive property compared to the conventional anionic urethane resin, since the cationic tertiary amino groups contained in the molecular chain interact with the negatively charged surface of the hair.

In addition, a particularly good washability can be achieved by adjusting a ratio of the carboxyl groups to the tertiary amino groups (a ratio between the numbers of respective functional groups) in the amphoteric urethane resin within a certain range.

## Claims

1. A cosmetic resin composition **characterized in that** it comprises as a major ingredient an amphoteric urethane resin having one or more carboxyl groups and one or more tertiary amino groups in a single molecule, wherein the amphoteric urethane resin is obtained by reacting the following Components (A) to (C) under the excessive presence of isocyanate groups to prepare a prepolymer containing the isocyanate groups, reacting said prepolymer containing the isocyanate groups with the following Component (D), and then blending the reactant with water containing an aliphatic polyamine to conduct chain-extending reactions:
(A) a polyol compound;
(B) a polyisocyanate compound;
(C) a compound having one or more active hydrogens and one or more carboxyl groups;
(D) a compound having one or more active hydrogens and one or more tertiary amino groups.

2. A cosmetic resin composition **characterized in that** it comprises as a major ingredient an amphoteric urethane resin having one or more carboxyl groups and one or more tertiary amino groups in a single molecule, wherein the amphoteric urethane resin is obtained by reacting the following Components (A), (B), and (D) under the excessive presence of isocyanate groups to prepare a prepolymer containing the isocyanate groups, reacting said prepolymer containing the isocyanate groups with the following Component (C), and then blending the reactant with water containing an aliphatic polyamine to conduct chain-extending reactions:
(A) a polyol compound;
(B) a polyisocyanate compound;
(C) a compound having one or more active hydrogens and one or more carboxyl groups;
(D) a compound having one or more active hydrogens and one or more tertiary amino groups.

3. The cosmetic resin composition according to Claim 1 or 2, which is a hair care composition.

4. The cosmetic resin composition according to any one of Claim 1 to 3, in which a ratio of the carboxyl groups to the tertiary amino groups (a ratio between the numbers of respective functional groups) in said amphoteric resin is carboxyl groups/tertiary amino groups = 1/10 to 10/1.

5. The cosmetic resin composition according to any one of Claim 1 to 4, in which said amphoteric urethane resin is dispersed or solubilized in water.

## Patentansprüche

1. Kosmetische Harzzusammensetzung, **dadurch gekennzeichnet, dass** sie als Hauptbestandteil ein amphoteres Urethanharz mit einer oder mehreren Carboxyl-Gruppen und einer oder mehreren tertiären Amino-Gruppen in einem einzelnen Molekül umfasst, wobei das amphotere Urethanharz durch Reaktion der folgenden Komponenten (A) bis (C) unter dem exzessiven Vorliegen von Isocyanat-Gruppen erhalten wird, um ein Präpolymer herzustellen, das die Isocyanat-Gruppen enthält, das die Isocyanat-Gruppen enthaltende Präpolymer mit der folgenden Komponente (D) zur Reaktion gebracht wird und anschließend der Reaktant mit Wasser gemischt wird, das ein aliphatisches Polyamin enthält, um Kettenverlängerungsreaktionen ablaufen zu lassen:
(A) eine Polyol-Verbindung;
(B) eine Polyisocyanat-Verbindung;
(C) eine Verbindung mit einem oder mehreren aktiven Wasserstoffatomen und einer oder mehreren Carboxyl-Gruppen;
(D) eine Verbindung mit einem oder mehreren aktiven Wasserstoffatomen und einer oder mehreren tertiären Amino-Gruppen.

2. Kosmetische Harzzusammensetzung, **dadurch gekennzeichnet, dass** sie als Hauptbestandteil ein amphoteres Urethanharz mit einer oder mehreren Carboxyl-Gruppen und einer oder mehreren tertiären Amino-Gruppen in einem einzelnen Molekül umfasst, wobei das amphotere Urethanharz durch Reaktion der folgenden Komponenten (A), (B) und (D) unter exzessivem Vorliegen von Isocyanat-Gruppen erhalten wird, um ein Präpolymer herzusteilen, das die Isocyanat-Gruppen enthält, das die Isocyanat-Gruppen enthaltende Präpolymer mit der folgenden Komponente (C) zur Reaktion gebracht wird, und der Reaktant mit Wasser gemischt wird, das ein aliphatisches Polyamin enthält, um eine Kettenverlängerungsreaktion ablaufen zu lassen:
(A) eine Polyol-Verbindung;
(B) eine Polyisocyanat-Verbindung;
(C) eine Verbindung mit einem oder mehreren aktiven Wasserstoffatomen und einer oder mehreren Carboxyl-Gruppen;
(D) eine Verbindung mit einem oder mehreren aktiven Wasserstoffatomen und einer oder mehreren tertiären Amino-Gruppen.

3. Kosmetische Harzzusammensetzung gemäß Anspruch 1 oder 2, die eine Haarpflegezusammensetzung ist.

4. Kosmetische Harzzusammensetzung gemäß mindestens einem der Ansprüche 1 bis 3, in der ein Verhältnis der Carboxyl-Gruppen zu den tertiären Amino-Gruppen (ein Verhältnis zwischen der Anzahl der jeweiligen funktionellen Gruppen) in dem amphoteren Harz in der Form vorliegt, das Carboxyl-Gruppen/tertiäre Amino-Gruppen 1/10 bis 10/1 ist.

5. Kosmetische Harzzusammensetzung gemäß mindestens einem der Ansprüche 1 bis 4, in der das amphotere Urethanharz in Wasser dispergiert oder solubilisiert ist.

## Revendications

1. Composition de résine cosmétique **caractérisée en ce qu'**elle comprend comme ingrédient principal une résine d'uréthanne amphotère ayant un ou plusieurs groupes carboxyle et un ou plusieurs groupes amino tertiaire dans la même molécule, dans laquelle la résine d'uréthanne amphotère est obtenue en faisant réagir les constituants (A) à (C) suivants en présence d'un excès de groupes isocyanate pour préparer un prépolymère contenant les groupes isocyanate, en faisant réagir ledit prépolymère contenant les groupes isocyanate avec le constituant (D) suivant, puis en mélangeant le corps réactionnel à de l'eau contenant une polyamine aliphatique pour conduire des réactions d'allongement de chaîne :
A - un polyol,
B - un polyisocyanate,
C - un composé ayant un ou plusieurs atomes d'hydrogène actifs et un ou plusieurs groupes carboxyle,
D - un composé ayant un ou plusieurs atomes d'hydrogène actifs et un ou plusieurs groupes amino tertiaire.

2. Composition de résine cosmétique **caractérisée en ce qu'**elle comprend comme ingrédient principal une résine d'uréthanne amphotère ayant un ou plusieurs groupes carboxyle et un ou plusieurs groupes amino tertiaire dans la même molécule, dans laquelle la résine d'uréthanne amphotère est obtenue en faisant réagir les constituants (A), (B) et (D) suivants en présence d'un excès de groupes polyisocyanate pour préparer un prépolymère contenant les groupes isocyanate, en faisant réagir ledit prépolymère contenant les groupes isocyanate avec le constituant (C) suivant, puis en mélangeant le corps réactionnel à de l'eau contenant une polyamine aliphatique pour conduire des réactions d'allongement de chaîne :
A - un polyol,
B - un polyisocyanate,
C - un composé ayant un ou plusieurs atomes d'hydrogène actifs et un ou plusieurs groupes carboxyle,
D - un composé ayant un ou plusieurs atomes d'hydrogène actifs et un ou plusieurs groupes amino tertiaire.

3. Composition de résine cosmétique suivant la revendication 1 ou 2, qui est une composition pour soins capillaires.

4. Composition de résine cosmétique suivant l'une quelconque des revendication 1 à 3, dans laquelle le rapport des groupes carboxyle aux groupes amino tertiaire (un rapport entre les nombres de groupes fonctionnels respectifs) dans ladite résine amphotère est un rapport groupes carboxyle / groupes amino tertiaire compris dans l'intervalle de 1/10 à 10/1.

5. Composition de résine cosmétique suivant l'une quelconque des revendications 1 à 4, dans laquelle ladite résine d'uréthanne amphotère est dispersée ou solubilisée dans de l'eau.
